# EUROPEAN PATENT SPECIFICATION

(11) **EP 2 265 248 B1**
(45) Date of publication and mention of the grant of the patent: **19.04.2017**
(21) Application number: 09723522.0
(22) Date of filing: 13.03.2009
(51) Int. Cl.: A61K 8/60, A61Q 19/00, A61Q 1/14, A61Q 19/10

(54) **USE OF ONE OR MORE SALTS OF GLYCYRRHIZIC ACID FOR REDUCING THE IRRITATING ACTION OF SURFACTANTS IN COSMETIC COMPOSITIONS**
VERWENDUNG EINES ODER MEHRERER GLYCYRRHIZINSÄURESALZE ZUR MINDERUNG DER REIZWIRKUNG VON TENSIDEN IN KOSMETISCHEN ZUSAMMENSETZUNGEN
UTILISATION D UN OU DE PLUSIEURS SELS DE L ACIDE GLYCYRRHIZIQUE POUR RÉDUIRE L ACTION IRRITANTE DES TENSIOACTIFS DANS LES COMPOSITIONS COSMÉTIQUES

(30) Priority: 17.03.2008 IT MI20080444
(43) Date of publication of application: 29.12.2010
(73) Proprietor: Giuliani S.p.A., 20129 Milano (IT)
(72) Inventor: MASCOLO, Antonio, I-20126 Milano (IT); BENEDUSI, Anna, I-20124 Milano (IT); GIULIANI, Giammaria, I-20123 Milano (IT)
(74) Representative: Appoloni, Romano
(86) International application number: PCT/EP2009/052961
(87) International publication number: WO 2009/115455

(56) References cited:
- FR-A- 2 753 096
- FR-A- 2 894 821
- GB-A- 2 072 014
- US-A1- 2005 136 085
- HESHAM R.OMAR,IRINA KOMAROVA,AHMED FATHY,RANIA RASHAD: "Licorice abuse : time to send a warning message", THERAPEUTIC ADVANCES IN ENDOCRINOLOGY AND METABOLIM, vol. 3, no. 4, 4 January 2012 (2012-01-04) , pages 125-138,

## Description

### FIELD OF THE INVENTION

The present invention relates to the use of an agent to combat and reduce the irritating action of surfactants in compositions to be applied to the skin for care or cleansing of the body.

### PRIOR ART

The ingredients of formulations for cleansing the body are in general surfactants, rheology modifiers, pH regulators, preservatives, pearling agents, chelating agents, antioxidants, perfumes and other substances with different functions.

The agents responsible for the cleansing action are surfactants, added for this purpose to the formulation in quantities generally of about or greater than 10%, compounds containing amphiphilic or amphipatic groups whose dual affinity owing to polar and non-polar substances underlies the cleansing mechanism. These surfactants present in high concentrations to perform the cleansing action are defined as primary surfactants, distinguished from other substances also with surfactant properties at times added in lesser concentrations, for example, to increase the persistence of the foam or the viscosity of the formulation or to improve other rheological properties thereof.

Specific classes of primary surfactants such as alkyl sulfates (AS), for example including dodecyl sulfate, and alkyl ether sulfates (AES), for example including sodium lauryl ether sulfate (SLES), are preferred as they produce soft, abundant and creamy foams, with a compact, homogeneous and persistent structure. However, it is known that these surfactants have a denaturing action on proteins and are therefore capable of modifying the barrier function of the skin, promoting the loss of transepidermal water, and of determining the onset of irritant contact dermatitis (ICD). The irritating action of these substances is dependent on the dose and method of application.

In this regard, cleansing formulations for the body can be divided into two categories related to contact time with the skin, i.e. products intended to perform their cleansing effect for a prolonged time (leave-on) and products intended to be removed by rinsing shortly after application (rinse-off).

Given the same contact surface, following application of a leave-on product there is an increase in the concentration of the substances present in the formulation on the skin, as a result of evaporation of the water through time; instead, with application of a rinse-off product, such as a bath foam, there is a reduction of this concentration on the skin as a result of dilution of the product with the wash water and also as a consequence of passage from solution or dispersed system of the product to foam, which with the contribution of an additional dispersion phase, air, causes a decrease in the concentration of the ingredients in contact with the skin.

In particular, there are also leave-on cleansing formulas such as cleansing and make-up removing lotions which do not require to be rinsed off the face after application. This product is usually applied using make-up remover pads or cotton wool. It is therefore normal for part of the formula to remain on the skin.

Often the cleansing/make-up removing action of these formulations is supported by occlusive substances, such as mineral oils or foaming products, which can alter the natural physiological loss of water through the skin, with consequent destructuring of the horny layer and opening of intracellular routes for substances with low molecular weight such as to cause noteworthy skin irritation and at times allergic reactions.

### SUMMARY OF THE INVENTION

The object of the present invention is to combat the irritating action on the skin produced by the chemical substances, primarily surfactants, included in formulations for cleansing of the body, both of the leave-on and rinse-off type.

### DETAILED DESCRIPTION OF THE INVENTION

Principally to solve this problem, but also to achieve other advantages which will be described below, the present invention proposes the use of an agent to combat and reduce the irritating action of alkyl sulfates and alkyl ether sulfates in compositions to be applied to the skin for care or cleansing of the face and body, characterized in that said agent is chosen from one or more salts of glycyrrhizic acid.

In particular, according to the invention said agent is chosen from one of the following salts of glycyrrhizic acid, or mixtures thereof: monoammonium glycyrrhizinate (MAG), triammonium glycyrrhizinate, monopotassium glycyrrhizinate, dipotassium glycyrrhizinate (DPG), tripotassium glycyrrhizinate, monosodium glycyrrhizinate, disodium glycyrrhizinate, trisodium glycyrrhizinate.

In order to better understand the characteristics and advantages of the invention, below are some non-limiting practical embodiments are described below with reference to compositions suitable for various applications.

The quantities of the ingredients in the compositions are expressed as percentage in weight/weight (w/w). The names of the chemical compounds are international nomenclature of cosmetic ingredients (INCI).

### Example 1

In a first embodiment of the invention the following ingredients were formulated to prepare a shower gel for sensitive skins
Water q.s. 100
Coco glucoside 10-15%
Disodium laureth sulfosuccinate 1-5%
Glycerin 1-5%
Sorbitol 1-3%
Monoammonium glycyrrhizate 2%
Sucrose cocoate 1-3%
Acrytates/C10-30 Alkyl Acrylate Crosspolymer 0.1-0.5%
Sodium hydroxymethylglycinate 0.5%
Parfum (Allergen Free) 0.5-1%
Lactic acid q.s. pH 6.0

### Example 2

In a further embodiment of the invention the following ingredients were formulated to prepare a normal shower gel
Water q.s. to 100 %
Sodium Laureth Sulfate (SLES) 9-15%
Ammonium Laureth Sulfate 1-3%
Disodium Cocoamphodiacetate 3-5%
Sodium Cocoyl Glutamate 3-6 %
Monoammonium glycyrrhizate 1.8 %
Dipotassium glycyrrhizate-0.2 %
PEG-150 Pentaerytrityl Tetrastearate 1-3%
PEG-6 Caprylic/Capric Glycerides 0,5-3%
Lactic acid q.s. pH 6.0
Metylparaben 0.05-0.2
Propylparaben 0.05-0.2
Phenoxyethanol 0.5-0.8%
Parfum (Allergen Free) 0.5-1%

### Example 3

In a further embodiment of the invention the following ingredients were formulated to prepare a facial cleansing mousse for sensitive skins
Sodium Cocoamphodiacetate 4-8%
Disodium laureth sulfosuccinate 5-10 %
Sodium chloride 0.3-0.8
Monoammonium glycyrrhizate 2.5 %
Sodium hydroxymethylglycinate 0.5%
Water q.s. to 100 %

### Example 4

In a further embodiment of the invention the following ingredients were formulated to prepare a facial cleanser for sensitive skins with osmoprotectants
Glycerin 1-5%
Betaine 0.1-1%
Taurine 0.1-1%
Inositol 0.1-1%
Xilitol 0.1-1%
Allantoin 0.05-0.4%
Monoammonium glycyrrhizate 2.2 %
Disodium cocoamphodiacetate 4-8%
Sodium methyl cocoyl taurate 1-7%
Disodium laureth sulfosuccinate 5-9%
Parfum (allergen free) q.s.
Pentylene glycol 5-8%
Caprylyl glycol 0.2-0.5%
Lactic acid q.s. to pH 5.5
Water q.s. to 100 %

### Example 5

In a further embodiment of the invention the following ingredients were formulated to prepare a leave-on type facial make-up removing composition
Isononyl isononanoate 4-8%
Isostearyl isostearate 3-7%
Isostearyl palmitate 0.3-4%
Sucrose tristearate 0.3-2%
Glyceryl stearate 0.1-0.8%
Monoammonium Glycyrrhizate 1.2 %
Dipotassium Glycyrrhizate 1.0%
Tetrasodium EDTA 0.2%
Xanthan gum 0.1-0.8%
Cocamidopropyl betaine 0.5-3%
Sucrose palmitate 2-6%
Glycerin 3-8%
Triethanolamine q.s. pH 6
Pentylene glycol 5-8%
Parfum q.s.
Water q.s. to 100%

### BRIIEF DESCRIPTION OF THE DRAWINGS

In order to experimentally assess the technical effect desired according to the objects of the invention, a study of in vitro skin irritation and of the relative activity performed by the salt monoammonium glycyrrhizinate (MAG) was conducted as described below and with reference to the figures of the appended drawings.
Figure 1 shows a diagram relative to an MTT viability test.
Figure 2 shows a diagram relative to an ELISA IL-1α test released in the maintenance medium, as described below.

### IN VITRO STUDY

### 1. PURPOSE

Compositions based on the surfactant sodium dodecyl sulfate (SDS) are examined. Maintaining the sodium dodecyl sulfate (SDS) as positive control of skin irritation at a fixed concentration of 5%, a series of solutions are prepared in sterile water with increasing doses of monoammonium glycyrrhizinate (MAG, titre > 98%).

Monoammonium glycyrrhizinate is also assessed as is at the concentrations of 2% and 5%.

### 2. EXPERIMENTAL DESIGN

### 2.1. ASSAY SYSTEM

2.1.1 EPISKIN™ model (EPISKIN-SM™), EPISKIN SNC Lyon, France, is a model of reconstructed human epidermis. The human keratinocytes are seeded on a dermal substitute consisting of a collagen type I matrix coated with type IV collagen. A highly differentiated and stratified epidermis model is obtained after a 13-day culture period comprising the basal, suprabasal, spinous and granular layers and a functional stratum corneum (Tinois et al., 1984). The model is used in the skin irritation applying the substances being examined at topical level to the epidermis and subsequently assessing cell viability.

Quality Control EPISKIN-SM kits were produced in accordance with the quality procedures (certified ISO 9001). All biological components of the epidermis and also the culture medium were tested for the presence of viruses, bacteria and mycoplasma. The quality of the final product was confirmed by a cytotoxicity assay (MTT test) with SDS and by histological examination.

The following solutions were prepared:

| COMPOSITION SOLUTIONS | SDS 5% + MAG 1% | SDS 5% + MAG 2% | SDS 5% + MAG 3% | MAG 2% | MAG 3% |
|---|---|---|---|---|---|
| CODE | 1 + MAG 1 | + MAG 2 | + MAG 3 | MAG 2 | MAG 5 |
| pH at 25 °C | 4.75 | 4.67 | 4.54 | 4.619 | 4.665 |

The quantities of the ingredients in the compositions are expressed as percentage in weight/weight.

### 2.2.1. EXPOSURE

10µl of the solutions were applied to the surface of the epidermis for 15 minutes, followed by 42 hours of recovery.

### 2.3 CONTROLS

| | NAME pH | IDENTIFICATION CODE | BATCH | MANUFACTURER |
|---|---|---|---|---|
| POSITIVE CONTROL | SDS 5% 5.80 | SDS 5% | 026K0201 | SIGMA L4509 |
| NEGATIVE CONTROL | STERILE WATER 7.19 | CN | NA | NA |

### 3. METHODS

VitroScreen is implementing a quality system in compliance with the GLP regulations (2004/9/CE and 2004/10/CE) in preparation of the GLP Audit and relative certification. The methods utilized in the study were entered in the list of VitroScreen methods.

Following a validation study coordinated by ECVAM the Episkin test system was validated by the ESAC (27/04/07); this uses a model of epidermis reconstructed in vitro - EPISKIN - to assess skin irritation produced by chemical substances for hazard identification and R38 classification: cell viability is quantified after 15 min of exposure + 42h of recovery. Quantification of IL-1α with the ELISA method is part of the validated method for skin irritation as it allows the substances to be distinguished even when there is no decrease in cell viability.

### 3.1 MTT ASSAY: CELL VIABILITY MEASUREMENT

### 3.1.1 Principle of the method

The MTT test is considered an international standard for quantification of cytotoxicity (ISO 10993-5). The method allows quantification of the cytotoxic effect produced by the product through measuring cell viability. Cytotoxicity is quantified by measuring the decrease in cell viability compared to an untreated negative control. It is based on the metabolizing reaction which takes place between the salt of tetrazolium (MTT) and the mitochondrial enzymes (succinate dehydrogenase) after contact with the product for the various treatment times: only the viable cell can transform the salt of tetrazolium into the insoluble derivative (Formazan), reaction which is highlighted by the formation of a purple color at the bottom of the insert.

Metabolization of the salt takes place at the level of the basal cells of the epithelium in the area above the polycarbonate support.

The purple colored derivative is extracted in isopropanol and quantified spectrophotometrically at 570 nm.

### 3.1.2 Procedure

At the end of treatment and post-incubation with the substances being examined, the EPISKIN models are transferred to 12 well plates, filled with 2 ml of MTT (used at the concentration of 0.3 mg/ml in the assay medium) and the test is conducted for 3 hours at 37°C, 5% CO2, 90% humidity.

The formazan is extracted from the tissue in Eppendorf tubes with 500 µl of acidified isopropanol (0.04 N HCL final concentration) with incubation in the dark under stirring for 4 hours at room temperature.

### 3.1.3 Materials

- Tetrazolium salt MTT (Sigma M2128)
- Isopropanol (Sigma)
- Assay Medium (Episkin)
- Phosphate Buffer Saline (PBS)
- Microplate Autoreader M-200 INFINITE (TECAN)
- Analytical balance XS204 Mettler (0.00001)

### 3.2 QUANTITATIVE DETERMINATION OF INTERLEUKIN IL-1 α

### 3.2.1 Principle of the method

Interleukin IL-1 α is known to be the principal modulator of events associated with the response of an inflammatory/irritative event: it is possible to dose release in the growth medium of epithelium and tissue reconstructed in vitro. This assay uses the ELISA technique. A monoclonal antibody specific for the specific interleukin is coated onto the wells of a 96 well plate. The standards and samples are inserted in the wells: where interleukin is present, this binds to the immobilized antibody. After washing the unbinded substances from the wells, a polyclonal antibody specific for the molecule being examined bound to an enzyme is added to the wells. After further washing to remove the unbound antibody-enzyme, a substrate solution is added to the plate and colors develop in proportion to the quantity of interleukin bound in the initial step. Color development is blocked with a specific solution and the intensity of color is measured Spectrophotometrically.

The results are expressed in pg/ml: the sensitivity range is 3.9 -250 pg/ml.

### 3.2.2 Procedure

At the end of the incubation period (42 hours) the medium samples are collected and preserved at -20°C in Eppendorf tubes. The samples are thawed at room temperature only before the test is conducted. The thawed solutions are stirred in a Vortex: then 200 µl is collected and inserted in the specific wells (triplicated) according to a pre-defined scheme.

### 3.2.3 Materials

Kit QUANTIKINE® (R &D systems) Code : DLA 50 (IL-1) Microplate Autoreader M200-INFINITE (TECAN)

### 4. PROTOCOL

### DAY 1: Receipt of the Episkin model

Upon arrival, the EPISKIN tissues are placed in a 6 well plate with 4 ml of PBS and the TEER is measured.

The EPISKIN tissues are then transferred to an incubator (37 °C, 5% CO2 and 90% humidity) in 12 well plates with maintenance medium and incubated for an entire night.

### DAY 2: Application of the substances being examined

The solutions are prepared and the pH is measured.

10µl of the substances being examined, of the positive (SDS 5%) and negative (sterile water) control are applied to the surface of the epidermis.

The treated tissues are maintained at room temperature for 15 minutes (± 0.5 minutes).

At the end of incubation the tissues are washed 2-3 times with PBS and placed in an incubator with fresh maintenance medium for 42 hours of recovery.

### DAY 4: MTT test

At the end of the incubation period the culture media are collected and preserved in cryovials at -20°C.

The TEER is measured post-treatment and the MTT test is then conducted for 3 hours at 37°C and formazan is subsequently extracted for 4 hours as described in the methods.

At the end of the test 2 x 200µl of sample for each well are transferred to 96 well plates and read spectrophotometrically at 570 nm.

### 5. DATA ACQUISITION

The optical densities are recorded directly by the Microplate autoreader (TECAN INFINITE M-200), exported to Excel files and the cell viability % is calculated compared to the optical density of the negative control.

### 5.1 INTERPRETATION OF THE RESULTS - PREDICTIVE MODEL

The irritation potential is determined by the viability value of the tissue exposed to the substance being examined.

EU Classification R38 is allocated to the substance which produces a viability of less than 50% compared to the negative control.

| | |
|---|---|
| Tissue VIABILITY ≤ 50 % | Irritant (I) R38 |
| Tissue VIABILITY > 50 % | Non-Irritant (N I) |

### 6. RESULTS

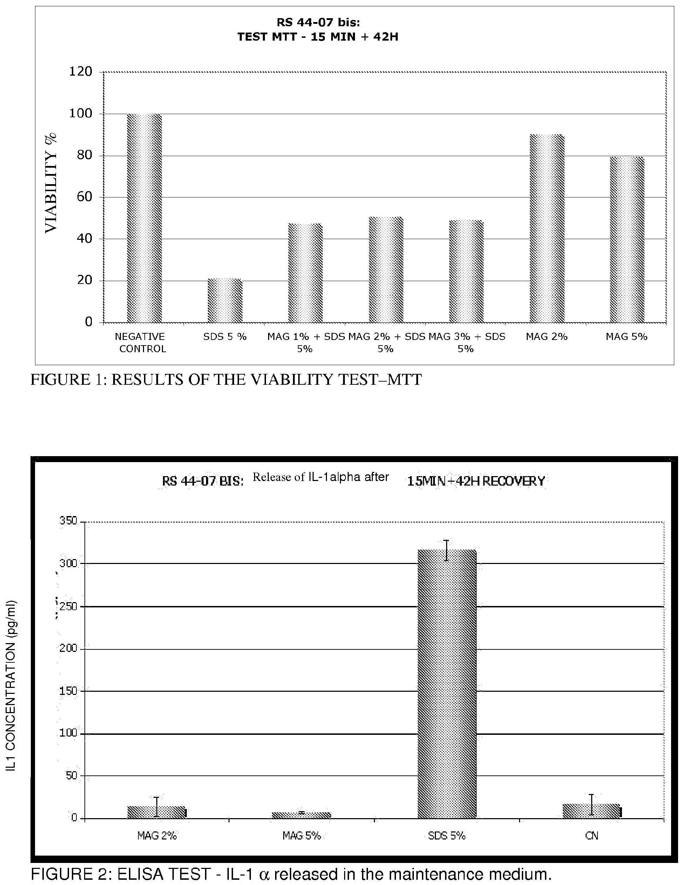

The results of the MTT test are indicated in Figure 1 of the appended drawing. The positive control SDS 5% produced a reduction in viability of 80% and the predictive model classifies it as R38 irritant, a result that allows validation of the assay.

The presence of MAG in the SDS solutions significantly reduced the cytotoxicity of SDS: the cell viability values of the samples containing increasing doses of MAG (1-2-3 %) are no different to one another and correspond to a viability ranging from 47% to 50%.

SDS The predictive model classifies the three products as borderline non-irritant (cut-off 50%).

The solution of MAG at 2% has no cytotoxic effects and at 5% a loss of viability of 20% is observed, which classifies both products as non-irritant.

The dose of IL-1 α implemented with the ELISA method is indicated in Figure 2 of the appended drawing.

No release is quantified in the negative control and a high release above the detectability limits (250 pg/ml) is instead detected for the positive control SDS 5%.

The MAG assessed at 2% and 5% did not produce any release of Interleukin -1 α, confirming the cell viability data.

### 7. CONCLUSIONS

Pro-inflammatory cytokine IL-1 α was measured.

The results classified as:
NON IRRITANT (NI) the Mono-Ammonium Glycyrrhizate tested at 2% and 5%.The dose of IL-1α in the culture medium confirmed the result.

IRRITANT the positive control SDS at 5%.

The solutions of SDS in the presence of graduated concentrations of MAG reduced the toxicity of the SDS by over 50% compared to the composition with SDS alone, giving viability values of 47% (MAG 1%), 49% (MAG 3%) and 50% (MAG 2%) with no significant differences from one another.

From the experimental study conducted, it is understood how the invention allows the objects set to the implemented effectively.

In a non-limiting manner, according to the present invention it is deemed that the ability of said agents to combat and reduce the irritating action of ionic surfactants, such as alkyl sulfates and alkyl ether sulfates, is due to the formation of mixed micelles of larger dimensions and with more rigid structure, consequently making their penetration more difficult.

Another aspect of the present invention is the ability of said agents, when added to leave-on cleansing compositions, to restore the barrier function of the skin while still supporting the cleansing function of the product.

In particular, they represent a surprising solution for the cleansing of sensitive skin, which from a physiological viewpoint is characterized by an almost constant inflammatory state at the level of the dermis.

Moreover, the present invention also represents an effective medium for carrying curative substances, such as anti-inflammatories, in products for care or cleansing of the body, as the compositions according to the invention do not have skin irritating properties even after prolonged periods of exposure, and therefore therapeutic or lenitive active ingredients can be added to them so that these ingredients remain on the skin for the time necessary to be effective.

## Claims

1. A salt of glycyrrhizic acid for use in reducing the irritating action of alkyl sulfates and alkyl ether sulfates as surfactants in compositions to be applied to the skin for care or cleansing of the face and body.

2. Salt according to claim 1, **characterized in that** it is chosen from one of the following salts of glycyrrhizic acid, or more than one of said salts to form mixtures thereof: monoammonium glycyrrhizinate (MAG), diammonium glycyrrhizinate, triammonium glycyrrhizinate, monopotassium glycyrrhizinate, dipotassium glycyrrhizinate (DPG), tripotassium glycyrrhizinate, monosodium glycyrrhizinate, disodium glycyrrhizinate, trisodium glycyrrhizinate.

3. Salt according to claim 1, **characterized in that** it is used in combination with a therapeutic or lenitive active ingredient.

4. Salt according to claim 1, **characterized in that** said surfactants comprise sodium lauryl ether sulfate (SLES).

5. Salt according to claim 1, **characterized in that** said surfactants comprise dodecyl sulfate.

6. Salt according to claim 1, **characterized in that** it is used in a composition in quantities ranging from 1 % to 3% by weight.

7. Composition for care or cleansing of the face and body comprising alkyl sulfates and alkyl ether sulfates as surfactants, **characterized in that** it comprises an agent to combat and reduce the irritating action of said surfactants, said agent being chosen from one or more of the following salts of glycyrrhizic acid: diammonium glycyrrhizinate, triammonium glycyrrhizinate, monopotassium glycyrrhizinate, dipotassium glycyrrhizinate (DPG), tripotassium glycyrrhizinate, monosodium glycyrrhizinate, disodium glycyrrhizinate, trisodium glycyrrhizinate.

8. Composition according to claim 7, **characterized in that** it contains one or more therapeutic or lenitive active ingredients.

9. Composition according to claim 7, **characterized in that** it contains said agent in quantities ranging from 1 % to 3% by weight.

## Patentansprüche

1. Salz der Glycyrrhizinsäure zur Verwendung bei der Verringerung der Reizwirkung von Alkylsulfaten und Alkylethersulfaten als Tenside in Zusammensetzungen zur Aufbringung auf der Haut zur Pflege oder Reinigung von Gesicht und Körper.

2. Salz nach Anspruch 1, **dadurch gekennzeichnet, dass** es ausgewählt ist aus einem der folgenden Salze der Glycyrrhizinsäure oder mehr als einem der Salze zur Bildung von Gemischen daraus: Monoammoniumglycyrrhizinat (MAG), Diammoniumglycyrrhizinat, Triammoniumglycyrrhizinat, Monokaliumglycyrrhizinat, Dikaliumglycyrrhizinat (DPG), Trikaliumglycyrrhizinat, Mononatriumglycyrrhizinat, Dinatriumglycyrrhizinat, Trinatriumglycyrrhizinat.

3. Salz nach Anspruch 1, **dadurch gekennzeichnet, dass** es in Kombination mit einem therapeutischen oder lindernden aktiven Bestandteil verwendet wird.

4. Salz nach Anspruch 1, **dadurch gekennzeichnet, dass** die Tenside Natriumlaurylethersulfat (SLES) umfassen.

5. Salz nach Anspruch 1, **dadurch gekennzeichnet, dass** die Tenside Dodecylsulfat umfassen.

6. Salz nach Anspruch 1, **dadurch gekennzeichnet, dass** es in einer Zusammensetzung in Mengen in einem Bereich von 1 Gew.-% bis 3 Gew.-% verwendet wird.

7. Zusammensetzung zur Pflege oder Reinigung von Gesicht und Körper umfassend Alkylsulfate und Alkylethersulfate als Tenside, **dadurch gekennzeichnet, dass** sie ein Mittel zur Bekämpfung und Verringerung der Reizwirkung der Tenside umfasst, wobei das Mittel aus einem oder mehreren der folgenden Salze von Glycyrrhizinsäure gewählt ist: Diammoniumglycyrrhizinat, Triammoniumglycyrrhizinat, Monokaliumglycyrrhizinat, Dikaliumglycyrrhizinat (DPG), Trikaliumglycyrrhizinat, Mononatriumglycyrrhizinat, Dinatriumglycyrrhizinat, Trinatriumglycyrrhizinat.

8. Zusammensetzung nach Anspruch 7, **dadurch gekennzeichnet, dass** sie einen oder mehrere therapeutische oder lindernde aktive Bestandteile enthält.

9. Zusammensetzung nach Anspruch 7, **dadurch gekennzeichnet, dass** sie das Mittel in Mengen in einem Bereich von 1 Gew.-% bis 3 Gew.-% enthält.

## Revendications

1. Sel d'acide glycyrrhizique destiné à être utilisé pour réduire l'action irritative de sulfates d'alkyle et de sulfates d'éther d'alkyle en tant que tensioactifs dans des compositions devant être appliquées sur la peau pour le soin ou le nettoyage du visage et du corps.

2. Sel selon la revendication 1, **caractérisé en ce qu'**il est choisi parmi un des sels d'acide glycyrrhizique suivants ou plusieurs desdits sels pour former des mélanges de ceux-ci : glycyrrhizinate de monoammonium (MAG), glycyrrhizinate de diammonium, glycyrrhizinate de triammonium, glycyrrhizinate de monopotassium, glycyrrhizinate de dipotassium (DPG), glycyrrhizinate de tripotassium, glycyrrhizinate de monosodium, glycyrrhizinate de disodium, glycyrrhizinate de trisodium.

3. Sel selon la revendication 1, **caractérisé en ce qu'**il est utilisé en combinaison avec un ingrédient actif thérapeutique ou lénitif.

4. Sel selon la revendication 1, **caractérisé en ce que** lesdits tensioactifs comprennent de l'éthersulfate sodique de lauryle (SLES).

5. Sel selon la revendication 1, **caractérisé en ce que** lesdits tensioactifs comprennent du dodécylsulfate.

6. Sel selon la revendication 1, **caractérisé en ce qu'**il est utilisé dans une composition dans des quantités allant de 1 % à 3 % en poids.

7. Composition destinée au soin ou au nettoyage du visage et du corps comprenant des sulfates d'alkyle et des sulfates d'éther d'alkyle en tant que tensioactifs, **caractérisée en ce qu'**elle comprend un agent pour combattre et réduire l'action irritative desdits tensioactifs, ledit agent étant choisi parmi un ou plusieurs des sels d'acide glycyrrhizique suivants : glycyrrhizinate de diammonium, glycyrrhizinate de triammonium, glycyrrhizinate de monopotassium, glycyrrhizinate de dipotassium (DPG), glycyrrhizinate de tripotassium, glycyrrhizinate de de monosodium, glycyrrhizinate de disodium, glycyrrhizinate de trisodium.

8. Composition selon la revendication 7, **caractérisée en ce qu'**elle contient un ou plusieurs ingrédients actifs thérapeutiques ou lénitifs.

9. Composition selon la revendication 7, **caractérisée en ce qu'**elle contient ledit agent dans des quantités allant de 1 % à 3 % en poids.
